# EUROPEAN PATENT SPECIFICATION

(11) **EP 1 018 340 B1**
(45) Date of publication and mention of the grant of the patent: **10.09.2003**
(21) Application number: 99670003.5
(22) Date of filing: 06.01.1999
(51) Int. Cl.: A61K 31/44, A61K 47/18, A61K 47/48, A61K 9/20, A61P 1/04

(54) **Inclusion aminoacid salts compounds of benzimidazole derivatives with cyclodextrins, their preparation and pharmaceutical formulations containing them**
Cyclodextrin Einschlusskomplexe mit Aminosäuresalzen von Benzimidazolderivaten, deren Herstellung, sowie diese enthaltende pharmazeutische Zusammensetzungen
Complexes d'inclusion de sels d'aminoacides de dérivés du benzimidazol et de cyclodextrines, leur préparation et formulations pharmaceutiques les contenant

(43) Date of publication of application: 12.07.2000
(73) Proprietor: TECNIMEDE-SOCIEDADE TECNICO-MEDICINAL, S.A., 2685-338 Prior Velho (PT)
(72) Inventor: Mendes Cerdeira, Ana Maria, 2700-098 Amadora (PT); De Sousa Goucha Jorge, Pedro Manuel, 2670-399 Loures (PT)
(74) Representative: Ferreira, Maria Silvina

(56) References cited:
- WO-A-96/38175
- WO-A-98/40069
- DATABASE CHEMICAL ABSTRACTS STN AN 124:211738, 15 April 1996 XP002107436 & HWANG ET AL.: "A comparative study on the pharmaceutical properties of rectal suppository containing omeprazole complexes" YACKCHE HAKHOECHI, vol. 25, no. 3, 1995, pages 227-237,

## Description

### Field of the Invention

This invention relates to pharmaceutical formulations containing a cyclodextrin inclusion compound of hydrosoluble salt of benzimidazole derivatives (amino acid salts) and its preparation by the simultaneous reaction of a cyclodextrin and an amino acid with a benzimidazole derivative.

### Background of the Invention

Omeprazole is a substituted benzimidazole, 5-methoxy-2-[[4-methoxy-3,5-dimethyl-2-pyridinyl)methyl]sulfinyl]-1H-benzimidazole, having the empirical formula C₁₇H₁₉N₃O₃S and a molecular weight of 354.42.

Omeprazole belongs to a class of compounds that reduce the gastric secretion, the substituted benzimidazoles. These compounds suppress the acidic secretion by specifically inhibiting the enzymatic system H+/K+ ATPase at the surface of the gastric parietal cells. As this enzymatic system was considered the proton pump inside the gastric mucosa, omeprazole was considered a proton pump inhibitor, because it blocks the final phase of the acid production. This drug is suitable for the short term treatment of the active duodenal ulcer, gastroesophageal reflow disease and long term pathological hypersecretory conditions, such as Zollinger-Ellison syndrome, et cetera.

However, discoloration and degradation is a characteristic of this group of molecules, such as omeprazole, when they are kept under acidic and neutral conditions. For example, omeprazole has a half-life shorter than 15 minutes at pH conditions. For example, omeprazole has a half-life shorter than 15 minutes at pH 4 and longer than 300 days at pH 11. These acid-unstable compounds also get degraded when they are in contact with moisture and organic solvents. (Matthew, M., and Bailey, R.E. (1995) Stability of Omeprazole Solutions at Various pH Values as Determined by High-Performance-Liquid-Chromatography. Drug. Dev. Ind. Pharm., 21, 965-971 and Pilbrant, A., Cederberg, *C.* (1985) Development of an Oral Formulation of Omeprazole. Scand. J. Gastroenterology, Suppl. 108, 113-120).

The oral administration of omeprazole requires therefore that this drug will be formulated in order to be protected against its acid and moisture degradation. Thus, the marketed formulations have a gastroresistant coating to prevent the omeprazole degradation inside the stomach.

EP 0 565 210 A2 describes the preparation of alkaline cores containing omeprazole, which are tabletted and coated with one or more layers of an inert under coating and one layer of an external enteric coating.

Canadian Patent Application A1 2 046 364 describes a galenic process to make omeprazole tablets, wherein an active coating having omeprazole, a protective coating and an external enteric coating are sprayed on inert cores.

WO 85/03436 describes a pharmaceutical formulation where the cores having one or more active ingredients mixed with buffering materials, such as sodium dihydrogen phosphate, are coated with a first coating which controls the diffusion in order to maintain a constant pH and a constant rate of diffusion. This formulation can not be adopted when using acid labile compounds where a quick release inside the small intestine is wanted. Direct application of an enteric coating onto the cores would adversely influence the storage stability of such dosage forms containing acid labile compounds.

EP 244380 refers to oral formulations containing labile compounds under acidic conditions having alkaline cores coated with gastroresistant polymers, specially sulfinylbenzimidazoles. The core is undercoated by one or more water soluble or water disintegrabl inert layers, the combination being coated with an enteric coating.

International Application PCT WO 94 02140 describes granules containing omeprazole that are tabletted so as to form tablet cores that afterwards are coated with three layers, the two first of which are buffering layers and the third one is an enteric layer.

International Application PCT WO 9623500 discloses stable galenic formulations containing a benzimidazole compound which is labile in acidic conditions/environment and their production: coated neutral cores of a water soluble polymer and compounds of non-alkaline reaction are prepared: afterwards a second isolating layer that comprises a water soluble polymer, a pigment and talc and finally an enteric-layer having one polymer, one plastifier and talc are applied.

However, due to the omeprazole instability or to the reaction of the drug with the free carboxylic groups of the used polymers in the gastroresistant coating, the omeprazole degradation takes place during the shelf period (Davidson, A.G., and McCallum, A (1996 ) A Survey of the Stability of Omeprazole Products from 13 countries. Drug Dev. Ind. Pharm., 22, 1173-1185), resulting in a low quality product for the patient.

Korean Patent 115254 refers to the use of a large excess of a basic amino acid as a form of stabilisation of omeprazole present in the core of a pharmaceutical formulation. However, this patent does not specify if the amino acid is used with the purpose of forming an omeprazole salt or as a simple physical mixture. Besides, the use of a large excess of amino acid (15 to 20 times the molar radio, amino acid/omeprazole) is economically disadvantageous.

US Patent 5232706 discloses a preparation having omeprazole or an omeprazole alkaline salt as a core (sodium or potassium salt for instance) and only an amino acid as a buffer agent. On the other hand, this patent does not present any stability data of the related formulations.

Japanese Patent 5194225 refers also to the association of a benzimidazole with an amino acid. However, these amino acids are used together with the buffer agents, acting as stabilisers, and the mechanism of their action is not explained. This patent only refers the stability data concerning the omeprazole coloration.

Korean Patent 9208161 B refers to stoichiometric omeprazole mixtures with amino acids (amino acid/omeprazole) of 0.1-5.0, and other conventional excipients, in order to obtain a stable core. However, according to Kook, K.C., *et al*, 1997 (Yakche Hakhoechi, 27, 1997), lifetime/shelf life at 20°C is 43 days for the stoichiometry (omeprazole : L-arginine) 1:15, 28 days for the 1:10 ratio and less than 10 days for a 1:5 ratio.

DE 3427785 A1 refers to inclusion compounds of cyclodextrins with sodium salts of benzimidazole derivatives. It is known that omeprazole sodium salts confer a high pH, being necessary to inform the users when they are used as an injectable formulation. Besides, sodium salts have the disadvantage of releasing sodium ions often not recommended

WO 93/13138 describes a process for the preparation of inclusion compounds of benzimidazole derivatives with cyclodextrins in basic medium where the alkaline salts used to buffer the medium are eliminated at the end of the reaction by washing them with water. The washing process may lead to the elimination of omeprazole and cause a low yield of the reaction. This process also includes heating the solution in order to obtain the complexes. Considering that omeprazole is a temperature sensible compound, this preparation process may produce degradation products.

WO 96/38175 discloses the formation of a cyclodextrin inclusion compound with a benzimidazole derivative by using a branched cyclodextrin having carboxyl groups, or carboxyl group salts, for injectable formulations. The production of this derivative is not very common, what may increase the price of the cyclodextrin. Those excipients are not economically feasible, excepting the β-cyclodextrin. Besides the economic reasons, β-cyclodextrin is the only one that is described in the pharmacopoeias.

WO98/40069 discloses that benzimidazoles can be stabilized by forming a complex with cyclodextrin in the presence of an amino acid.

The pharmaceutical compositions containing omeprazole that are included in the state of the art can be divided in three groups: compositions containing omeprazole alone, without any stabilising agent; compositions containing omeprazole and stabilising agents such as sodium salts and amino acids; and compositions containing omeprazole and cyclodextrins. However, the compositions that only have omeprazole inside the core or those having omeprazole and one amino acid in a molar ratio omeprazole/amino acid under 1:10 have some stability problems (Davidson, A., G., and McCallum, A. (1996) A survey of the stability of omeprazole products from 13 countries. Drug. Dev. Ind. Pharm., 22, 1173-1185 and Kook, K., C., et al (1997) Yakche Hakhoechi, 27). Besides, the compositions containing omeprazole and amino acids require very expensive industrial formulation steps such as the solvent evaporation under reduced pressure. On the other hand, all these processes only allow to prepare powdered materials that have to be further processed in order to obtain the pharmaceutical formulations.

### Summary of the Invention

It is an object of the present invention to simultaneously obtain an inclusion compound of a benzimidazole derivative with one or more amino acids and one or more cyclodextrins, and a pharmaceutical formulation.

The present invention discloses the reaction of a benzimidazole derivative with one or more amino acids followed by the reaction with one or more cyclodextrins, as a process to obtain an inclusion complex of a salt of a benzimidazole derivative.

The present invention also comprises the production of stable pharmaceutical formulations of benzimidazole derivatives, specially omeprazole, lansoprazole and pantoprazole. It is necessary that these formulations will be stable during the shelf period and, when given to patients, will have good properties of dissolution, and absorption, and accordingly an adequate bioavailability.

### Brief Description of the Drawings

The following description of the preferred process for obtaining the inclusion complexes of the present invention is supported by the enclosed NMR (nuclear magnetic resonance) and DSC (differential scanning calorimetry) spectra as shown by the drawings of the enclosed figures, wherein
- Fig. 1,: shows the NMR spectrum of omeprazole in DMSO (300 MHz);
- Fig. 2,: the NMR spectrum of omeprazole L-arginine salt in DMSO (300 MHz);
- Fig. 3,: the DSC spectrum of omeprazole;
- Fig. 4,: the DSC spectrum of L-arginine;
- Fig. 5,: the DSC spectrum of omeprazole L-arginine salt (ratio 1/5);
- Fig. 6,: the NMR spectrum of β-cyclodextrin in DMSO (300 MHz);
- Fig. 7,: the NMR spectrum of the complex omeprazole/L-arginine/β-cyclodextrin 1/5/1 in DMSO (300 MHz);
- Fig. 8,: the NMR spectrum of the complex omeprazole/L-arginine/β-cyclodextrin in DMSO (300 MHz);
- Fig. 9,: the NMR spectrum of the complex omeprazole/L-arginine/β-cyclodextrin 1/5/3 in DMSO (300 MHz);
- Fig. 10,: the DSC spectrum of β-cyclodextrin;
- Fig. 11,: the DSC spectrum of the complex omeprazole/L-arginine/β-cyclodextrin 1/5/1;
- Fig. 12,: the DSC spectrum of the complex omeprazole/L-arginine/β-cyclodextrin 1/5/2;
- Fig. 13,: the DSC spectrum of the complex omeprazole/L-arginine/β-cyclodextrin 1/5/3;
- Fig. 14,: the DSC spectrum of the complex omeprazole/L-arginine/β-cyclodextrin 1/5/2 being present in granules for extrusion/spheronization.

### Detailed Description of the Invention

The present invention has the object of obtaining a cyclodextrin inclusion compound of a benzimidazole derivative salt with an amino acid. In this way, the benzimidazole derivative and the amino acid are associated to at least a cyclodextrin, as a mixture of these compounds, a mixture of the benzimidazole derivative salt and a cyclodextrin or the inclusion compound of the benzimidazole derivative/amino acid/cyclodextrin.

The amino acid to be used is a basic amino acid in the molar ratio (benzimidazole derivative:cyclodextrin) range from 1:1 to 1:5, preferentially 1:2.

Following a preparation method, the amino acid (L-arginine) is solubilized in water or in an ethanol:water mixture; the drug is added to this solution. The obtained solution or suspension is mixed at high speed during 1 to 10 minutes. The acid-base reaction for preparing the benzimidazole derivative salt with a basic amino acid, more specifically the benzimidazole derivative salt with L-arginine, is carried out according to one of with the following schematic reactions:
Omeprazole 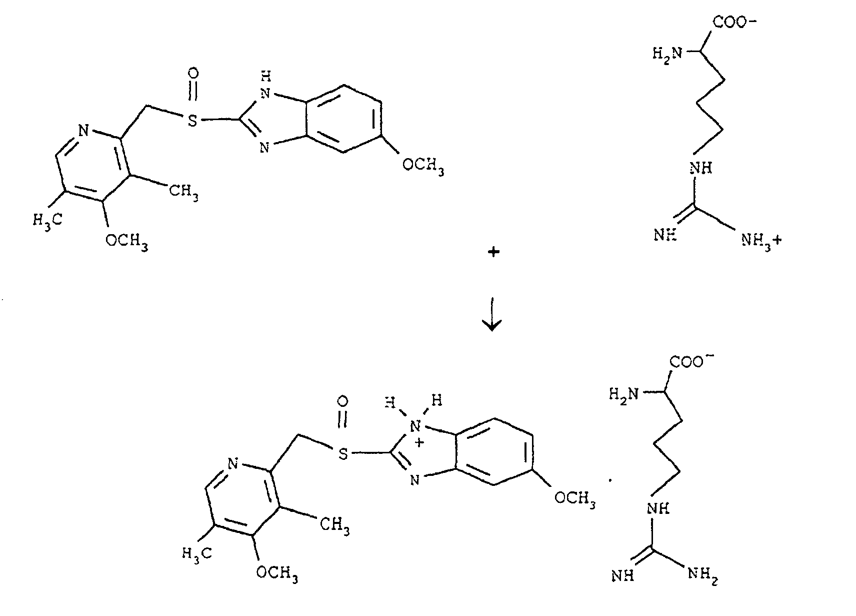
Lansoprazole 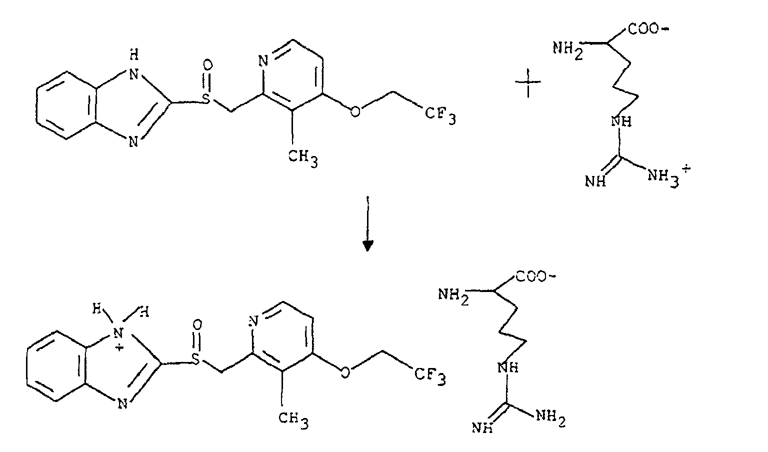
Pantoprazole

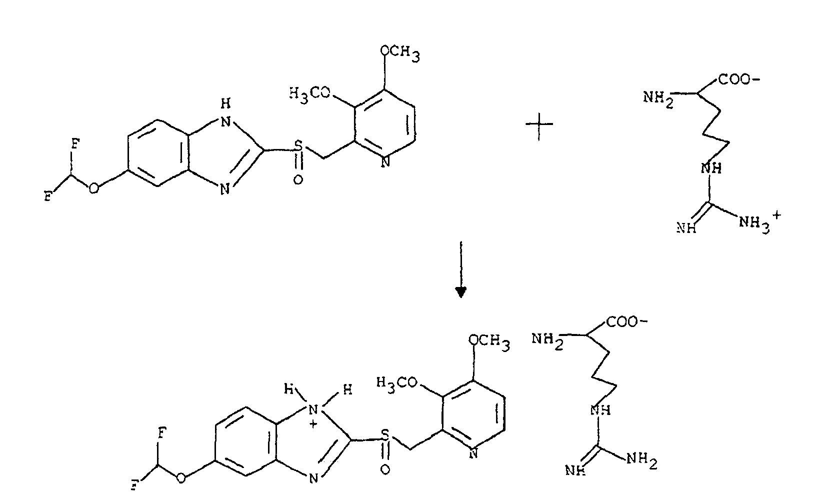

The obtained benzimidazole derivative salts are subsequently complexed with the cyclodextrin.

The operating conditions of the above mentioned reaction are as follows: the amino acid is dissolved in water or in an ethanol: water mixture maintained at room temperature, preferentially not being higher than 40°C. The drug is than added to this solution or suspension, producing a solution or suspension, when it is used an ethanol:water mixture or water. The stirring period may vary from 1 to 10 minutes, since the acid-base reaction is very quick. After this period of time, β-cyclodextrin is added maintaining a strong agitation, during from 0.5 to 24 hours, more preferentially 0.5 to 12 hours, or 2 to 2.5 hours.

The amino acid and the cyclodextrin react together and protect the benzimidazole derivative against the several types of degradation that these compounds tend to sustain, namely the degradation that occurs when the drug contacts the free carboxylic groups of the gastroresistant polymers.

From cyclodextrins, β-cyclodextrin is preferentially used. The molar ratio of benzimidazole derivative/L-arginine/β-cyclodextrin advantageously varies from 1/5/1 to 1/5/3.

These benzimidazole derivatives based associations are finally prepared by spray-drying, lyophylisation, solvent evaporation under reduced pressure, by deposition on inert cores or by a granulation processe to obtain tablets or pellets by extrusion-spheronization.

The following non limitative examples illustrate several ways used to obtain solid inclusion compounds containing the benzimidazole derivative salt and the cyclodextrin and to simultaneously obtain pharmaceutical formulations.

### Example 1: Preparation of omeprazole/L-arginine salts and their inclusion compounds with β-cyclodextrin

### Stoichiometry 1/5 - Omeprazole/L-arginine

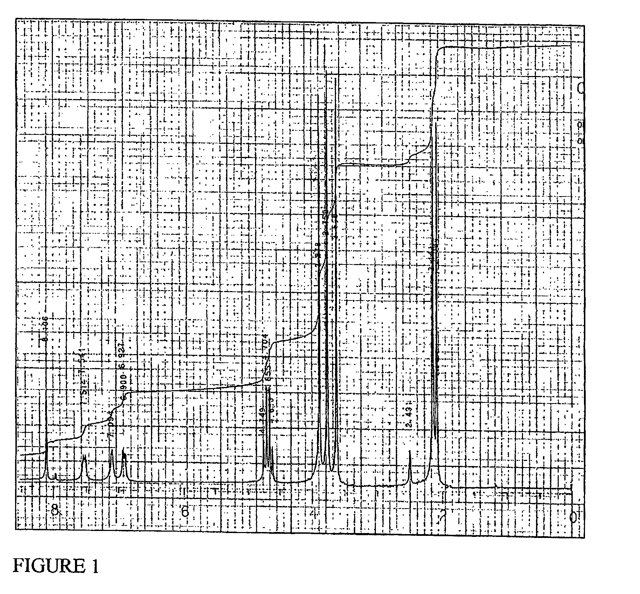

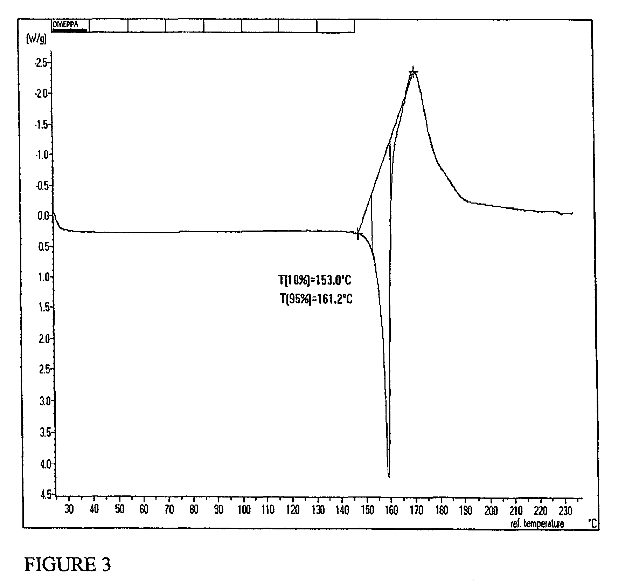

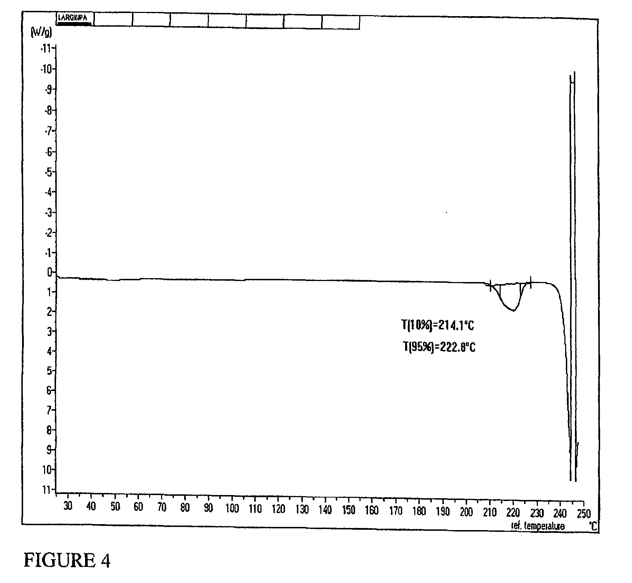

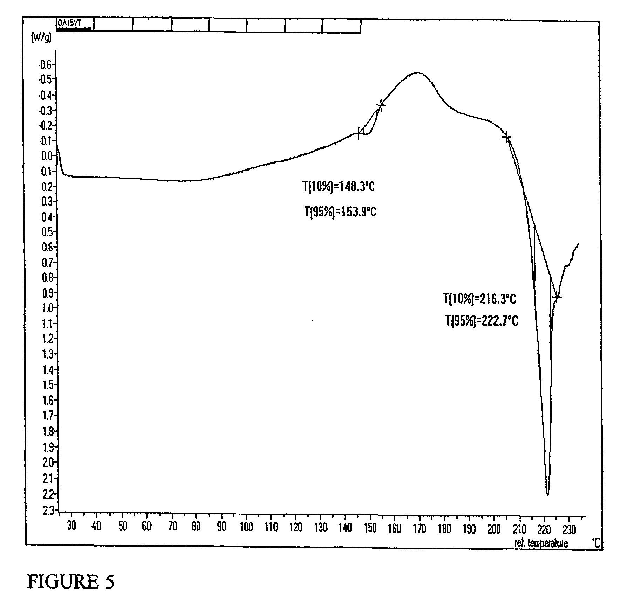

L-arginine (4.9 g) and omeprazole (2.0 g) were dissolved in 200 ml of an ethanol:water mixture (30:70). After solvent evaporation by the spray-drying process, differential scanning calorimetry (DSC) and nuclear magnetic resonance (NMR) studies were performed. The corresponding plotted spectra are shown in Fig 1 to 5.

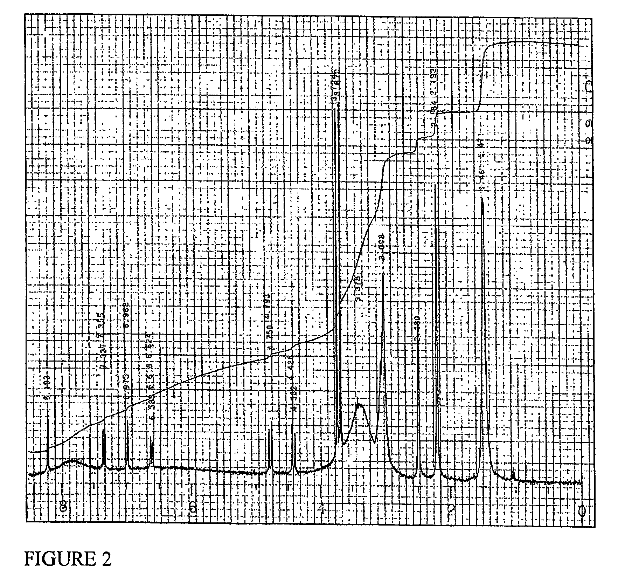

As it can be observed in Figure 2, the band corresponding to the chemical shift of 8 ppm suggests that omeprazole was salified.

The DSC spectra also suggest the formation of an omeprazole salt, since the melting point peak of omeprazole disappeared, which may mean the formation of a new identity.

**Stoichiometry of 1/5/1, 1/5/2 and 1/5/3 - Omeprazole/L-arginine/β-cyclodextrin** L-arginine (0.888 g) was dissolved in an ethanol: water mixture. Omeprazole (0.362 g) was added. After complete dissolution of both compounds, 1.307 g, 2.613 g or 3.920 g of β-cyclodextrin were added. The solution was spray dried.

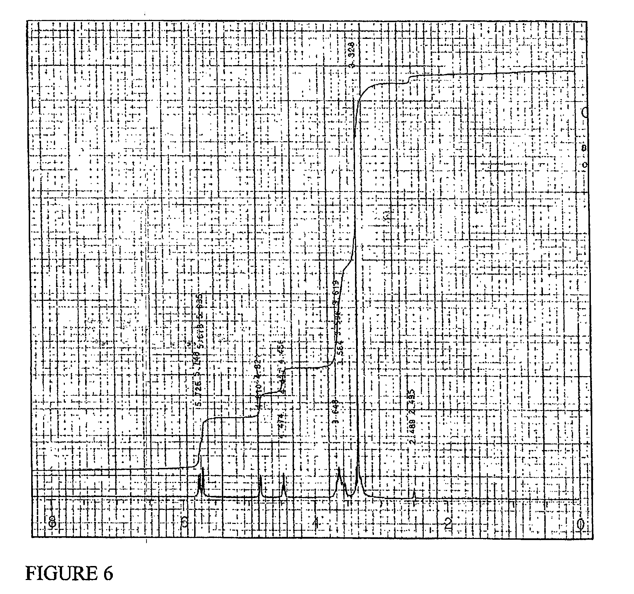

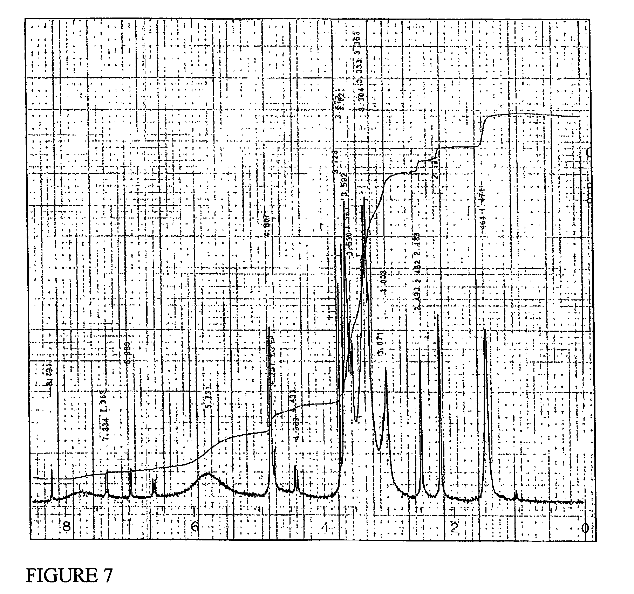

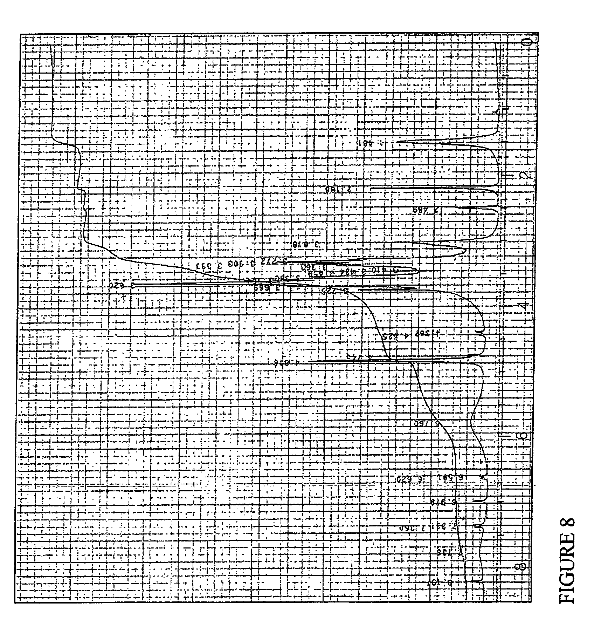

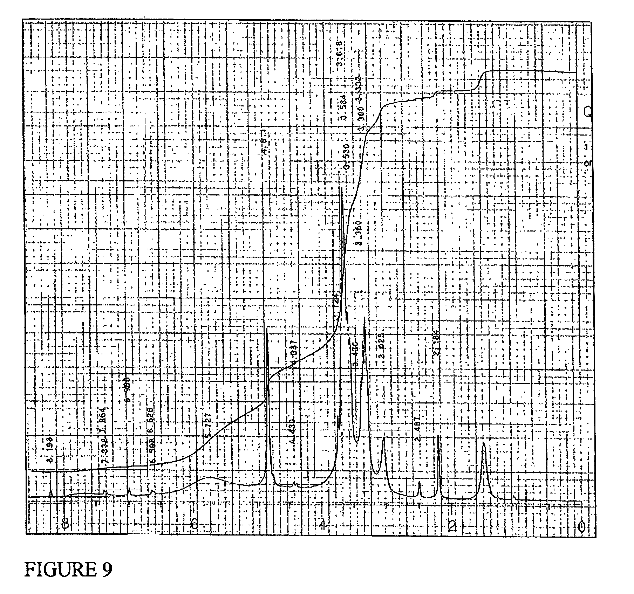

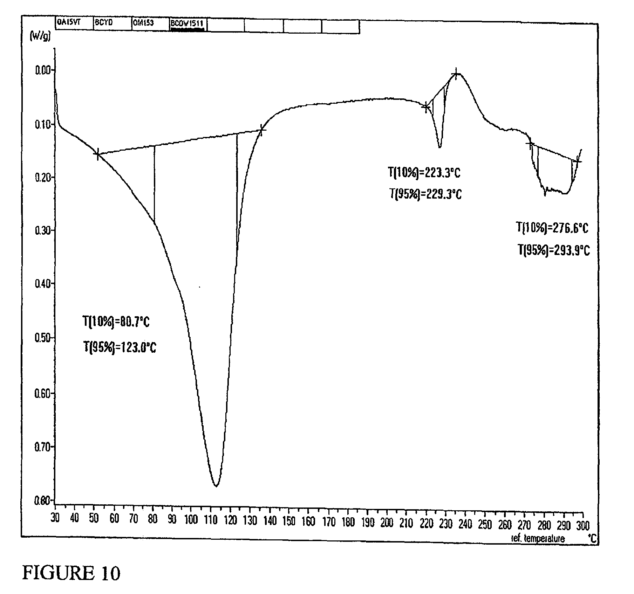

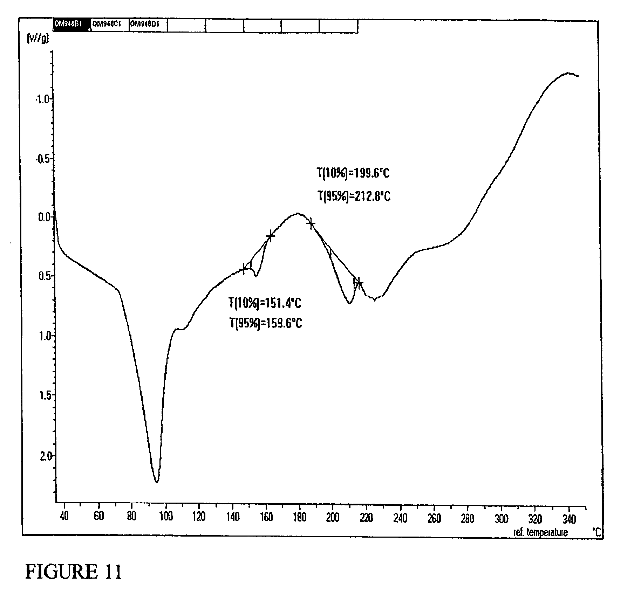

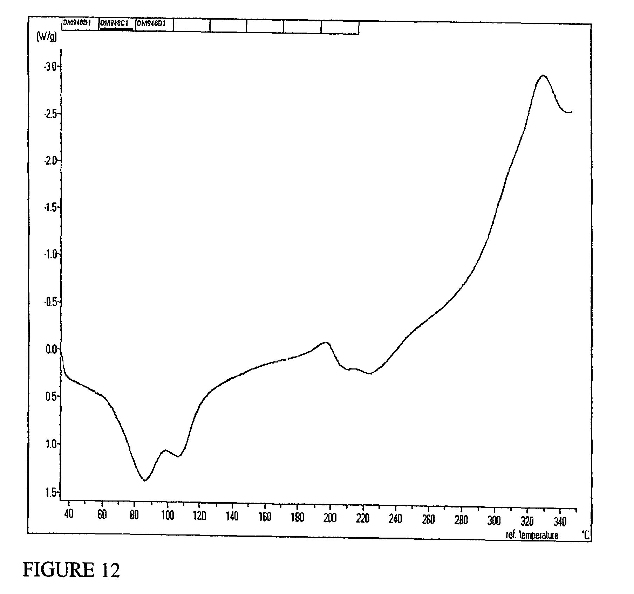

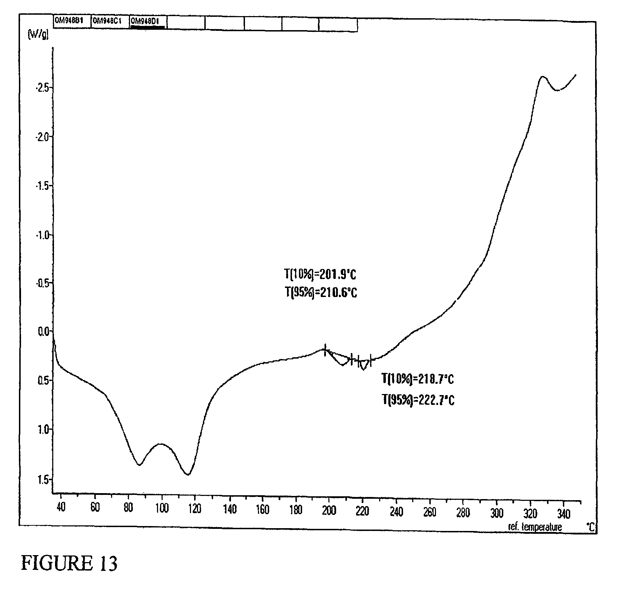

Their NMR and DSC spectra were plotted. The enclosed Fig. 6 to 13 show these spectra.

The differences between the chemical shifts of the cyclodextrin and the benzimidazole derivative salt are due to the formation of an inclusion compound, with special importance to the 1/5/2 and 1/5/3 stoichiometries. These results are also in accordance with the DSC spectra, where it can be observed that the melting point of omeprazole disappeared.

### Example 2: Stability studies of the omeprazol/L-arginine/β-cyclodextrin inclusion compounds

### Inclusion compound preparation

L-arginine (7.4 g) was added to 200 ml of water. Omeprazole (3.0 g) was than added to this suspension under vigorous stirring. After a stirring period of 5 minutes, 10.84 g, 21.68 g or 32.54 g of β-cyclodextrin were added, according to the stoichiometries 1/5/1, 1/5/2 or 1/5/3. The suspension stirring was maintained for 2 hours at 6000 rpm. After the water evaporation by lyophylization, the resulting inclusion compounds were kept at 25°C, 60% RH (relative humidity) and at 40°C, 75% RH. The omeprazole content of the inclusion compounds of the above described samples was determined after exposure periods of 7 and 60 days. The obtained results are presented in Tables 1, 2 and 3.

**Table 1 -**

| Omeprazole content at time 0 in the inclusion compounds having the molar ratios of 1/5/1, 1/5/2 and 1/5/3 | |
|---|---|
| | Omeprazole content (%) (n=2 ± s.d.) |
| Stoichiometry (OMP.:L-arg.: β-cyd.) | 25°C, 60% RH |
| 1/5/1 | 98.27 ± 0.56 |
| 1/5/2 | 97.18 ± 0.49 |
| 1/5/3 | 101.24 ± 1.38 |
| OMP. - omeprazole; L-arg. - L-arginine; β-cyd. - β-cyclodextrin; RH - relative humidity; s.d. standard deviation | |

**Table 2 -**

| Omeprazole content after an exposure period of 7 days of the inclusion compounds having molar ratios of 1/5/1, 1/5/2 and 1/5/3 | | |
|---|---|---|
| | Omeprazole content (%) (n=2 ± d.p.) | |
| stoichiometry (OMP.:L-arg.: β-cyd.) | 25°C, 60% RH | 40°C, 75% RH |
| 1/5/1 | 98.66 ± 0.37 | 98.93 ± 0.00 |
| 1/5/2 | 99,16 ± 0.62 | 99.16 ± 0.90 |
| 1/5/3 | 96.15 ± 0.83 | 99.08 ± 0.56 |
| OMP. - omeprazole; L-arg. - L-arginine; β-cyd. - β-ciclodextrin; RH - relative humidity; s.d. standard deviation | | |

**Table 3 -**

| Omeprazole content after an exposure period of 60 days, of the inclusion compounds having molar ratios of 1/5/1, 1/5/2 and 1/5/3 | | | | |
|---|---|---|---|---|
| | Omeprazole content (%) (n=2 ± d.p.) | | Degradation products (%) | |
| Stoichiometry (OMP.:L-arg.: β-cyd.) | 25°C, 60% RH | 40°C, 75% RH | 25°C, 60% RH | 40°C, 75% RH |
| 1/5/1 | 100.42 ± 0.23 | 96.64 ± 0.87 | n.d. | 1.45 |
| 1/5/2 | 101.13 ± 0.07 | 98.59 ± 0.59 | n.d. | 0.80 |
| 1/5/3 | 100,22 ± 0.57 | 97,61 ± 0,04 | n.d. | 0.84 |
| OMP. - omeprazole; L-arg. - L-arginine; β-cyd. - β-cyclodextrin; RH - relative humidity; n.d. not detected; s.d. standard deviation | | | | |

As it can be observed in Table 3, after an exposure period of 60 days at 40°C, 75% RH, the inclusion compounds of omeprazole/L-arginine/β-cyclodextrin are still stable with special significance for the stoichiometries 1/5/2 and 1/5/3. The degradation products are within the limits imposed by European Pharmacopoeia.

### Example 3: Enteric coated tablets containing an inclusion compound of omeprazole/L-arginine/β-cyclodextrin, prepared by direct compression

| ***Inclusion compound preparation*** | |
|---|---|
| | Composition (g) |
| Omeprazole | 20.0 |
| β-cyclodextrin | 142.6 |
| L-arginine | 49.0 |
| Water | 1108.4 |

### - Preparation method

L-arginine was dissolved in 1108.4 g of purified water. Omeprazole was then added under stirring. After a stirring period of 5 minutes, β-cyclodextrin was suspended in the last. The suspension stirring was maintained for 2 hours at 6000 r.p.m. After the water evaporation, the inclusion compound was used for tablet preparation.

| ***Preparation of 10 mg omeprazole tablets*** | |
|---|---|
| Composition (g) | |
| Omeprazole inclusion compound* | 86.8 |
| Microcrystalline cellulose | 213.0 |
| Colloidal silica | 3.0 |
| Magnesium stearate | 3.0 |

| | |
|---|---|
| * Corresponding to 10 mg of omeprazole | |

The tablets were obtained by direct compression.

| ***Enteric coating of tablets*** | |
|---|---|
| Composition (g) | |
| Eudragit L 30D-55 | 1082.4* |
| Triethyl citrate | 39.7 |
| Talc | 64.0 |

| | |
|---|---|
| *Corresponding to 324.7 g of polymer | |

The enteric coating was applied by the film-coating tecnique.

### Tablet analysis

The omeprazole content was assayed (HPLC) in the tablet cores and in the enteric coated tablets, at time 0 (Table 4) and after a period of 60 days without wrapping package at 40°C, 75% RH (Table 5). In Table 5 are also given the results of the omeprazole content for the tablets maintained at the temperature of 25°C, 60% RH.

### Gastro-resistance studies

The gastro-resistance studies were performed according to USP 23 (2 hours in 0.1N HCl, 37°C). The remaining omeprazole and degradation products contents were evaluated by HPLC (Table 6).

**Table 4 -**

| Omeprazole content in the tablet cores and in the enteric coated tablets (time 0) | |
|---|---|
| | Omeprazole content (%) (n=2 ± d.p.) |
| | 25°C, 60% RH |
| Cores | 108.1 ± 1.1 |
| Enteric coated tablets | 95.7 ± 3.5 |

**Table 5 -**

| Omeprazole content in cores and enteric coated tablets after an exposure period of 60 days. | | | | |
|---|---|---|---|---|
| | Omeprazole content (%) (n=2 ± d.p.) | | Degradation products (%) (n=2) | |
| | 25°C, 60% RH | 40°C, 75% RH | 25°C, 60% RH | 40°C, 75% RH |
| Cores | 99.0 ± 3.9 | 104.6 ± 6.3 | -- | -- |
| Tablets | 101.4 ± 6.3 | 95.3 ± 3.0 | n.d. | 6.8 ± 0.2 |
| n.d. not detected | | | | |

**Table 6 -**

| Omeprazole tablet content after 2 hours in 0.1N HCl | |
|---|---|
| **Omeprazole content (%)** | 102.2 ± 2.5 |
| Degradation products (%) | n.d. |
| n.d. not detected | |

The results of the omeprazole content determination made on the enteric coated tablets without any protective coat between the omeprazole core and the enteric coat, stored at 40°C, 75% RH without any wrapping, show that after 60 days in adverse conditions of moisture and temperature, the amount of degraded compounds is lower than the provided by other commercial products, the stability test of which was performed in the commercial package (Davidson, A., G., and McCallum, A. (1996) A survey of the stability of omeprazole products from 13 countries. Drug. Dev. Ind. Pharm., 22, 1173-1185).

The in vitro release studies have also shown the gastroresistance of the tablets, since there was no omeprazole release to the dissolution media, the upper limit of 10% being imposed by US Pharmacopeia. This assay also revealed the stability of tablets in acidic media, since after 2 hours of contact with 0.1 HCl solution no degradation compound was detected.

### Example 4: Preparation of omeprazole/L-arginine/β-cyclodextrin tablets by a wet granulation process

| ***Preparation of 20 mg omeprazole tablets*** | |
|---|---|
| | Composition (g) |
| Omeprazole | 20.0 |
| L-arginine | 49.2 |
| β-cyclodextrin | 145.0 |
| Microcrystalline cellulose | 196.7 |
| Sodium croscarmellose | 36.8 |
| Magnesium stearate | 2,3 |

The tablets were prepared by wet granulation, using a suspension containing omeprazole, L-arginine and β-cyclodextrin.

Same composition, except for magnesium stearate, was used to obtain pellets by extrusion-spheronization.

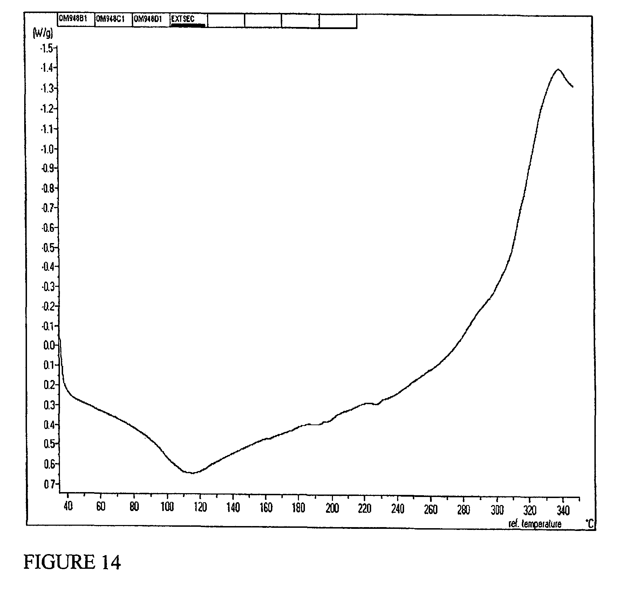

Figure 14 shows the DSC spectrum of the inclusion compound present in the mass for extrusion/spheronization, and proofs that the simultaneous obtention of a solid complex and a pharmaceutical formulation is possible.

To study the tablet stability, 2 batches of tablet cores were produced, one of them containing only an gastro-resistant enteric coat and the other containing an enteric coat and a protective coat (hydroxypropyl methylcellulose) between the tablet core and the latter. The tablets including the cores were kept at 25°C, 60% RH and at 40°C, 75% RH, without any wrapping. The omeprazole content was evaluated at time 0 (HPLC) and after 15 days. The obtained results are described in Tables 7 and 8.

**Table 7 -**

| Omeprazole content in the coated tablets (time 0) | |
|---|---|
| | Omeprazole content (%) (n=2 ± s.d.) |
| | 25°C, 60% RH |
| Coated tablets (enteric coat) | 100.5 ± 0.66 |
| Coated tablets (enteric coat + protective coat) | 100.2 ± 2.69 |

**Table 8 -**

| Omeprazole content in the coated tablets (time 15 days) | | | |
|---|---|---|---|
| | Omeprazole content (%) (n=2 ± s.d.) | | Degradation products (%) (n=2) |
| | 25°C, 60% RH | 40°C, 75% RH | 40°C, 75% RH |
| Coated tablets (enteric coat) | 102.9 ± 1.44 | 100.31 ± 0.72 | 2.12 |
| Coated tablets (enteric coat + protective coat) | 101.1 ± 0.59 | 103.54 ± 0.10 | 1.75 |

## Claims

1. A stable inclusion compound of a benzimidazole derivative amino acid salt and a cyclodextrin, obtainable by the process comprising the steps:
a. dissolving the amino acid in water or in an ethanol:water-mixture;
b. adding the benzimidazole derivative to this solution;
c. mixing the solution at high speed for 1 to 10 minutes;
d. adding a cyclodextrin to the solution obtained in step c while maintaining a strong agitation from 0.5 to 24 hours;
e. preparing the final stable inclusion compound by a method selected from spray drying, lyophilisation, solvent evaporation under reduced pressure, by deposition on inert cores or by a granulation process, to obtain tablets or pellets by extrusion-spheronization.

2. An inclusion compound according to claim 1, **characterised in that** the benzimidazole derivative is omeprazole, lansoprazole or pantoprazole.

3. An inclusion compound according to claim 1, **characterized in that** the basic amino acid is L-arginine, L-lysine, histidine or any other alkaline amino acid.

4. An inclusion compound according to claim 1, **characterised in that** the cyclodextrin is β-cyclodextrin, γ-cyclodextrin, α-cyclodextrin, their hydrates, derivatives or their mixtures.

5. An inclusion compound according to claim 4, **characterised in that** the used cyclodextrin derivative is selected from the alkyl or hydroxyalkyl derivatives.

6. An inclusion compound according to claims 1 to 5, **characterised in that** benzimidazole derivative/amino acid/cyclodextrin molar ratios are from 1/1/1 to 1/10/4.

7. Process of preparing the inclusion compound according to claims 1 to 6, **characterised in that** said three compounds are reacted in an aqueous or hydroalcoholic solution and afterwards the solvent is vaporized.

8. Pharmaceutical formulations **characterised in that** they contain the inclusion compound according to claims 1 to 6.

9. Solid pharmaceutical formulations according to claim 8, **characterised in that** they are coated with an enteric polymer, namely a polymeric derivative of methacrylic acid, or polyvinyl acetate phthalate.

10. Use of the formulations according to claim 9, in the manufacture of a medicine for the prophylactic and therapeutic treatment of duodenal gastric ulcer, gastroesophageal reflow disease and long term hypersecretory conditions, such as Zollinger-Ellison syndrome.

## Patentansprüche

1. Stabiles Cyclodextrin Einschlusskomplex mit einem Aminosäuresalz von Benzimidazolderivaten, erhältich durch ein Verfahren, das aus folgenden Schritten besteht:
a. die Aminosäure im Wasser oder in einem Ethanol:Wasser-gemisch lösen;
b. das Benzimidazolderivat dieser Lösung hinzugeben;
c. die Lösung unter hoher Geschwindigkeit während 1 bis 10 Minuten mischen;
d. das Cyclodextrin der in Schritt c erhaltenen Lösung hinzugeben, wobei eine starke Bewegung durch Rühren während 0,5 bis 24 Stunden erhalten wird;
e. Vorbereitung des stabilen Endeinschlusskomplexes nach einer Methode gewählt aus Sprühtrocknung, Lyophilisation, Lösungsmittelverdampfung unter vermindertem Druck, durch Ablagerung auf inerte Kerne oder durch einen Granulationprozeß, um Tabletten oder Pellets durch Extrusion-Sphäronisierung zu erhalten.

2. Einschlusskomplex gemäß Anspruch 1, **dadurch gekennzeichnet, daß** das Benzimidazolderivat Omeprazol, Lanoprazol oder Pantoprazol ist.

3. Einschlusskomplex gemäß Anspruch 1, **dadurch gekennzeichnet, daß** die basische Aminosäure L-Arginin, L-Lysin, Histidin oder irgendeine andere alkalische Aminosäure ist.

4. Einschlusskomplex gemäß Anspruch 1, **dadurch gekennzeichnet, daß** das Cyclodextrin β-Cyclodextrin, γ-Cyclodextrin, α-Cyclodextrin, seine Hydrate, Derivate oder seine Mischungen ist

5. Einschlusskomplex gemäß Anspruch 4, **dadurch gekennzeichnet, daß** das benutzte Cyclodextrinderivat aus Alkyl- oder Hydroxyalkylderivaten gewählt wird

6. Einschlusskomplex gemäß den Ansprüchen 1 bis 5, **dadurch gekennzeichnet, daß** die Molverhältnisse von Benzimidazolderivat/Aminosäure/Cyclodextrin von 1/1/1 bis 1/1014 betragen.

7. Verfahren zur Herstellung des Einschlusskomplexes gemäß den Ansprüchen 1 bis 6, **dadurch gekennzeichnet, daß** die drei gennanten Verbindungen in eine wäßrige oder hydroalkoholische Lösung reagiert werden, und danach das Lösungsmittel verdampft wird.

8. Pharmazeutischen Formulierungen, **dadurch gekennzeichnet, daß** sie einen Einschlusskomplex gemäß Ansprüchen 1 bis 6 enthalten.

9. Feste pharmazeutische Formulierungen gemäß Anspruch 8, **dadurch gekennzeichnet, daß** sie mit einem enteralen Polymer, nämlich einem Polymerderivat einer Methacrylsäure oder Polyvinylazetat phthatalat überzogen sind.

10. Verwendung der Formulierungen gemäß Anspruch 9, zur Herstellung eines Arzneimittels für die prophylaktische und therapeutische Behandlung des gastroduodenalen Geschwüres, der gastroösophagealer Reflux-Krankheit und langfristiger hypersekretorische Erkrankungen, wie Zollinger-Ellison Syndrom.

## Revendications

1. Complexe d'inclusion stable d'un sel d'aminoacide dérivé du benzimidazole et une cyclodextrine, obtenu par le procédé comportant les étapes:
a. dissolution de l'aminoacide dans l'eau ou dans une mixture d'éthanol:eau;
b. addition du dérivé de benzimidazole à cette solution;
c. mélange de la solution à grande vitesse pour 1 à 10 minutes;
d. addition d'une cyclodextrine à la solution obtenue en étape c pendant que se maintient une agitation forte de 0,5 à 24 heures;
e. préparation du complexe d'inclusion stable final par une méthode choisie parmi le séchage par atomisation, lyophilisation, évaporation du dissolvant à pression réduite, par le dépôt sur des noyaux inertes ou par un procédé de granulation, pour obtenir des comprimés ou pellets par extrusion-spheronisation.

2. Complexe d'inclusion selon la revendication 1, **caractérisé en ce que** le dérivé de benzimidazole est omeprazole, lanoprazole ou pantoprazole.

3. Complexe d'inclusion selon la revendication 1, **caractérisé en ce que** l'aminoacide basique est L-arginine, L-lysine, histidine ou n'importe quel autre aminoacide alcalin.

4. Complexe d'inclusion selon la revendication 1, **caractérisé en ce que** la cyclodextrine est β-cyclodextrine, γ-cyclodextrine, α-cyclodextrine, leurs hydrates, dérivés ou leurs mélanges.

5. Complexe d'inclusion selon la revendication 4, **caractérisé en ce que** le dérivé de cyclodextrine utilisé est choisi parmi les dérivés alkyliques ou hydroxyalkyliques.

6. Complexe d'inclusion selon les revendications 1 à 5, **caractérisé en ce que** les rapports molaires du dérivé benzimidazole/aminoacide/cyclodextrine sont de 1/1/1 à 1/10/4.

7. Procédé pour préparer le complexe d'inclusion selon les revendications 1 à 6, **caractérisé en ce que** lesdits trois composés sont fait réagir dans une solution aqueuse ou hydroalcoolique et après le dissolvant est vaporisé.

8. Formulations pharmaceutiques **caractérisées en ce qu'**elles contiennent le complexe d'inclusion selon les revendications 1 à 6.

9. Formulations pharmaceutiques solides selon la revendication 8, **caractérisées en ce qu'**elles sont enduites avec un polymère entérique, à savoir un dérivé polimérique de l'acide méthacrylique, ou phtalate d'acétate de polyvinyle.

10. Utilisation des formulations selon la revendication 9, pour la fabrication d'un médicament pour le traitement prophylactique et thérapeutique de l'ulcère gastro-duodénal, de la maladie de reflux gastro-oesophagien et des états hypersécrétoires de longue durée, telles que le syndrome de Zollinger-Ellison.
